# EUROPEAN PATENT APPLICATION

(11) **EP 4 506 023 A1**
(43) Date of publication of application: **12.02.2025**
(21) Application number: 24174097.6
(22) Date of filing: 03.05.2024
(51) Int. Cl.: A61M 1/36, A61M 25/00

(54) **CANNULAE FOR EXTRACORPOREAL CIRCULATION**

(30) Priority: 05.05.2023 WO PCT/CN2023/092219; 05.01.2024 CN 202410029026
(71) Applicant: ChinaBridge (Shenzen) Medical Technology Co., Ltd., Shenzhen, Guangdong 518101 (CN)
(72) Inventor: BONGERT, Markus, Shenzhen, 518101 (CN); PHILIPP, Alois, 93080 Pentling (DE)
(74) Representative: Pfenning, Meinig & Partner mbB

(57) **Abstract**

The present invention relates to cannulae for extracorporeal circulation of blood. More specifically, it relates to cannulae used to access the vessels of a patient either to deliver or to drain blood for circulating and processing it outside the body. In particular, it relates to arterial and venous cannulae for extracorporeal circulation having slotted openings.

## Description

### CROSS REFERENCE TO RELATED APPLICATIONS

The present application is a continuation of and claims priority to PCT/CN2023/092219, internationally filed on May 5, 2023, and claims priority to Chinese Patent Application No. 202410029025.2, filed January 9, 2024, both of which are incorporated herein by reference.

### TECHNICAL FIELD

This disclosure relates to the field of devices for extracorporeal circulation of blood. More specifically, it relates to cannulae used to access the vessels of a patient either to deliver or to drain blood for circulating and processing it outside the body.

### BACKGROUND

Cannulae are an important component of an extracorporeal blood circulation system, for example, in connection with a cardiopulmonary bypass procedure. Cannulae are used to draw deoxygenated blood from a patient's heart, direct the blood through components of a bypass system (e.g., an oxygenator), and return oxygenated blood to the heart. One of such procedures is extracorporeal membrane oxygenation (ECMO), which includes various modalities of temporary mechanical cardiopulmonary assistance of the failing heart and/or lungs. It's established to provide systemic perfusion and gas exchange, allowing the heart and lungs to rest and recover during a heart surgical intervention or bridge the patient to another modality of mechanical support or transplantation. Particularly, ECMO procedures may last up to several days, if not a few weeks, during which the extracorporeal system must properly and continuously support the patient. ECMO procedures involve redirecting the blood from the body through cannulae and connecting tubing to a gas-exchange membrane oxygenator and then returning it, by means of a pump, back to the patient's natural circulation system. Several modes of ECMO are available for advanced heart/lung failure. The exact configuration of the ECMO circuit and cannulation strategy depends on the organ(s) to be supported. In case of severe heart, lung or combined heart-lung failure, access to a patient's circulatory system may be obtained for establishing a peripheral ECMO by percutaneous cannulation of femoral artery, femoral vein or internal jugular vein, or a central ECMO, in which right heart and aorta are cannulated.

Cannulae, therefore, are key elements in extracorporeal circulation, including ECMO, and must provide the least traumatic, most durable and simplified method of delivering the blood to and from the patient. Despite several different solutions already available in the field, there remains the need of cannulae with improved performances for the patient and better ease of use for the operator.

### SUMMARY

In Example1, a cannula for conveying blood in an extracorporeal circulation system includes: a tubular body having a proximal portion having a first diameter and a distal portion having a second diameter smaller than the first diameter, the tubular body defining an internal lumen extending from a proximal end to a distal end along an axis, the distal end including a distal opening The distal portion of the tubular body defines at least two rows of side holes in fluid communication with the internal lumen, a first row disposed proximal to the distal opening and a second row spaced in a proximal direction along the axis from the first row, the side holes of each row having a rectangular shape with a length L and a width W. The side holes of the first row have a first length L and a first width W and the side holes of the second row have a second length L and a second width W, each of which are less than the first length L and the first width W. The L/W ratio of each side hole is between 2.3 and 2.7. Example 1 applies mainly to cannulae for blood perfusion. Compared to a design where the distal portion of the cannula consists of entirely circular holes with or without reducing L/W ratio , Example 1 side holes design shows a favorable flow characteristic, increases side hole flow, balances pressure loss, reduces velocity gradient of the hole flow and prevents either blood stagnation or lower limb ischemia, particularly in case of substantial loss of perfusion due to cannula occlusion.

Example 2 is the cannula of Example 1 wherein the first row includes a first side hole and the second row includes a second side hole, each of the first row and second row including only a single side hole.

Example 3 is the cannula of Example 1 wherein the first and second rows are spaced apart at a distance of about 7 to 9 mm in the proximal direction.

Example 4 is the cannula of Examples 2 wherein the first side hole and the second side hole are circumferentially opposed.

Example 5 is the cannula of Examples 1-4 further comprising a third row having a third side hole and a fourth row having a fourth side hole, the third row spaced in a proximal direction along the axis from the second row and the fourth row spaced in a proximal direction along the axis from the third row wherein the side hole third row have a third length L and a third width W each of which are less than the second length and the second width W and the side holes of the fourth row have a fourth length L and a fourth width W, each of which are less than the third length L and the third width W.

Example 6 is the cannula of Example 5 further wherein each of the first row, second row, third row and fourth row are spaced equally from each other at a distance of from about 7 to about 9 mm.

Example 7 is the cannula of Example 5 wherein each of the third row and fourth row include only a single side hole.

Example 8 is the cannula of Examples7 wherein the first and third side holes are substantially circumferentially aligned, the second and fourth side holes are substantially circumferentially aligned, and the first and third side holes are generally circumferentially opposed to the second and fourth side holes.

Example 9 is the cannula of Example 5 wherein the second length, third length and fourth length are each progressively reduced by about one sixth of the first length and further wherein the second width, third width and fourth width are each progressively reduced by about one sixth of the first width.

Example 10 is the cannula of Examples 9 wherein the fourth length is about one half the first length and the fourth width is about one half the first width.

Example 11 is the cannula of any of the preceding Examples wherein the L/W ratio of each side hole is 2.5.

In Example 12, a cannula for conveying blood in an extracorporeal circulation system includes: a tubular body having a proximal portion having a first diameter and a distal portion having a second diameter smaller than the first diameter, the tubular body defining an internal lumen extending from a proximal end to a distal end along an axis, the distal end having a distal opening. The distal portion of the tubular body defines a first set of three rows of side holes in fluid communication with the internal lumen, of which a first row is disposed proximal to the distal opening, a second row is spaced in a proximal direction along the axis from the first row, and a third row is spaced in a proximal direction along the axis from the second row, each of the side holes having a rectangular shape with a length L and a width W. Each row of the first set has at least two side holes and wherein the side holes of the first row have a first length L and a first width W and the side holes of the second row have a second length L and a second width W and the side holes of the third row have a third length L and a third width W the second length and width being less than the first length and width and the third length and width being less than the second length and width.The L/W ratio of each side hole is between 1.3 and1.7. Example 12 applies mainly to cannulae for blood drainage. Example 12 side holes design shows favorable flow characteristics, mainly with respect to a more evenly distributed flow, pressure and velocity profile through all side holes compared to other shapes.

Example 13 is the cannula of Example 12 wherein the side holes of each of the first, second and third rows are circumferentially opposed and further wherein each of the side holes of the first row are circumferentially aligned with each of the side holes of the third row.

Example 14 is the cannula of Example 13 wherein the first row, second row, and third row are each equally spaced from each other.

Example 15 is the cannula of Examples 14 wherein each of the first row, second row and third row are equally spaced from each other at a distance of from about 48 to 52 mm.

Example 16 is the cannula of Example 14 wherein each of the first row, second row and third row are equally spaced from each other at a distance of from about 23 to 27 mm.

Example 17 is the cannula of Example 15 further including a second set of two single side hole rows disposed between the distal opening and the first row of the first set, including a fourth row disposed distal to a fifth row, wherein the fifth row is spaced from the first row of the first set at a distance of from about 44 to 48 mm.

Example 18 is the cannula of Example 16 further including a second set of two single side hole rows disposed between the distal opening and the first row of the first set, including a fourth row disposed distal to fifth row, wherein the fifth row is spaced from the first row of the first set at a distance of from about 19 to 23 mm.

Example19 is the cannula of Examples 17 to 18 wherein the side hole lengths of the second set are equal to each other and wherein the side hole widths of the second set are equal to each other and wherein the L/W ratio of the second set side holes is 1.3 to 1.7. The staggered configuration of these single side hole rows optimizes the axial-symmetrical flow function, while directing the flow away from the cannula tip and helping to reduce the likelihood of damage to the vessel wall.

Example 20 is the cannula of Example 19 wherein the first length, second length and third length of the first set of side holes are each progressively reduced by about one sixth of the second set length, and wherein the first width, second width and third width of the first set of side holes are each progressively reduced by about one sixth of the second side hole width.

Example 21 is the cannula of Examples 17 to 18 wherein the fourth row and the fifth row are spaced from each other at a distance of from about 3 to 6 mm.

Example 22 is the cannula of Examples 12 to 21 wherein the L/W ratio of each side hole is 1.5.

Example 23 is the cannula of Examples 17 to 22 further including a sixth row of side holes disposed between the fourth row of the second set and the distal opening. Moreover, the sixth row has at least two side holes and serves the purpose of evenly distributing the pressure and velocity profile along the cannula, thereby allowing a gradual inflow acceleration across the cannula. The absence of such holes disposed proximal to the distal opening would result in a regional concentration of flow, pressure, shear stress and velocity distribution at the distal portion of the cannula, thereby increasing residence time, stagnation, likelihood of thrombi formation and hemolysis within the cannula.

Example 24 is the cannula of Example 23, wherein the sixth row is spaced along the axis in a distal direction from the fourth row at a distance of from about 39 to 43 mm.

Example 25 is the cannula of Examples 23 to 24 where the sixth row of side holes is spaced along the axis in a proximal direction from the distal opening at a distance of from about 8 to 12 mm.

Example 26 is the cannula of Examples 23 to 25 wherein, the fourth row of side holes, the fifth row of side hole and the sixth row of side holes all have the same length and width.

Example 27 is the cannula of Examples 23 to 26, wherein third row of side holes have a length and a width about 50 percent less than the length and width of the fourth, fifth and sixth row of side holes.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 shows a schematic view of a peripheral venous-arterial extracorporeal membrane oxygenation (VA-ECMO) circulatory support system operatively coupled to a patient.
FIG. 2a shows a top side view and FIG. 2b shows a bottom (i.e., 180 degrees apart from FIG. 2a) side view of a perfusion cannula according to various embodiments of the present invention.
FIG. 3 shows an exemplary slotted side hole according to embodiments of the present invention.
FIGS. 4a-4c show various views of a drainage cannula according to various embodiments of the present invention.

While the invention is amenable to various modifications and alternative forms, specific embodiments have been shown by way of example in the drawings and are described in detail below. The intention, however, is not to limit the invention to the embodiments described. On the contrary, the invention is intended to cover all modifications, equivalents, and alternatives falling within the scope of the invention as defined by the appended claims.

### DETAILED DESCRIPTION

The following detailed description is exemplary in nature and is not intended to limit the scope, applicability, or configuration of the invention in any way. Rather, the following description provides some practical illustrations for implementing exemplary embodiments of the present invention. Examples of constructions, materials, and/or dimensions are provided for selected elements. Those skilled in the art will recognize that many of the noted examples have a variety of suitable alternatives.

As the terms are used herein with respect to measurements (e.g., dimensions, characteristics, attributes, components, etc.), and ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although illustrative methods may be represented by one or more drawings (e.g., flow diagrams, communication flows, etc.), the drawings should not be interpreted as implying any requirement of, or specific order among or between, various steps disclosed herein. However, certain some embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

Cannulae are medical devices having a tubular body typically constructed from polymeric materials, including for example, polyurethane or PVC. The cannula body extends from a proximal end (near the operator) to a distal end (distant from the operator), which are connected by a lumen extending through all or most of the length of the tubular body. The cannulae body typically includes open ends at its extremities, a distal opening, also referred to as a tip, which is inserted into the patient vessel and a proximal opening, which is connected to the extracorporeal circuit. The cannula body is often divided into two different axial portions having different diameters, i.e., a proximal portion and a distal portion. The diameter of the distal portion defines the cannulae size and is typically smaller than the diameter of the proximal portion. The two portions are joined together by a tapered (e.g., intermediate conical) section. As the blood vessels have different diameters, cannulae are available in different sizes, e.g., smaller for perfusion (arterial) than for drainage (venous). In a perfusion cannula, blood travels from the proximal to the distal direction, whereas in a drainage cannula, blood travels in the opposite direction (i.e., from the distal to the proximal).

According to various embodiments, perfusion (i.e., arterial) cannulae diameters range from 14 to 19 French (Fr) and lengths range from about 20 to 40 cm. In certain embodiments, the length of the perfusion cannula is about 30 cm. According to various embodiments, drainage (venous) cannulae diameters range from about 19 to about 25 French and lengths up to 70 cm. Cannulae, as previously mentioned, must be sufficiently flexible to ease introduction and positioning into the patient's vessels. Moreover, cannulae must be sufficiently resistant to kinking and to permanent deformations that would otherwise cause internal lumen reductions and flow/pressure drop alterations. Further, a cannula must provide, for a given pressure drop, a sufficient flow rate therethrough, or, for a given flow rate, a sufficiently low pressure drop, which in both cases creates a favorable flow-pressure drop profile and limits the mechanical stress on blood elements (with consequent hemolysis, cell activation and coagulation).

To achieve these performance characteristics, in certain embodiment, the cannulae have sizes (outer diameters) as close as possible to the cannulated vessel diameters and wall thicknesses reduced to a few tenths of a millimeter. In some embodiments, the wall thickness is between 0.3 and 0.6 mm, and in some embodiments, the wall thickness is between about 0.4 and 0.5 mm, and in some embodiments, the wall thickness is about 0.48 mm.

To ensure sufficient cannula mechanical strength, in certain embodiments, appropriate metal spirally wound wires (i.e., coils) are embedded along the cannula length to provide the necessary resistance, combined with flexibility (not otherwise obtainable by an extremely thin polymeric tube alone). In such embodiments, the metal coil acts like a sort of cannula strong and flexible reinforcement keeping the lumen cylindrical and open to blood passage, avoiding kinking when the cannula is subjected to bending during introduction and positioning. The coil may be embedded into the cannula polymeric tube by several fabrication technologies, like dipping, lamination, etc.

The cannula tip is an open end traversed by the main blood stream. In certain embodiments, the distal portion of the cannula includes a structure of side holes adapted to provide an optimal fluid-dynamic pattern to blood delivered to the patient (in perfusion cannulae), or, vice versa, in collecting and removing blood from the patient (in drainage cannulae). The total surface area of such side holes is typically substantially larger than that of the tip opening and, in addition to influencing the cannula flow/pressure drop characteristics, the side holes may provide several beneficial effects. For perfusion cannulae, the side holes reduce the flow rate ejected by the tip, which helps avoid damaging the internal surface of the vessel. Further, the side holes may enable a certain amount of perfusion flow (i.e., flow in the opposite direction to the main flow) to the lower limbs (e.g., to prevent their ischemia), or, for drainage cannulae, they may contrast blood stagnation and recirculation areas along the distal portion, thus preventing localized clot formation and improving efficiency of operation. The number, shape, reciprocal distance and positioning of the side holes along the distal portion is important to ensure efficient cannula performance.

According to various embodiments, the side holes are positioned along the outer surface of the cannula distal portion and are organized in one or more rows. As used herein, a "row" of side holes indicates one or more side holes positioned substantially the same distance from the cannula tip. In various embodiments, the cannula includes multiple rows of side holes, wherein at least one row has multiple distinct side holes and a least one other row has a single side hole. In various embodiments, one or more of the side holes have a "slotted" configuration, which indicates that the holes have an elongated (i.e., have a length L greater than a width W) rectangular shape. In some embodiments, the elongated side holes have square corners and in other embodiment the elongated side holes have rounded corners.

In some embodiments, the elongated side holes of the same cannula are also characterized by the same ratio between their individual length L and width W (i.e. L/W), which may range from 1.2 to 3 according to various embodiments as detailed below. Moreover, L and W values for holes in the same row may be either equal or decreased by quantities (kept fixed throughout the same cannula) as compared to L and W values of the adjacent row in the distal direction.

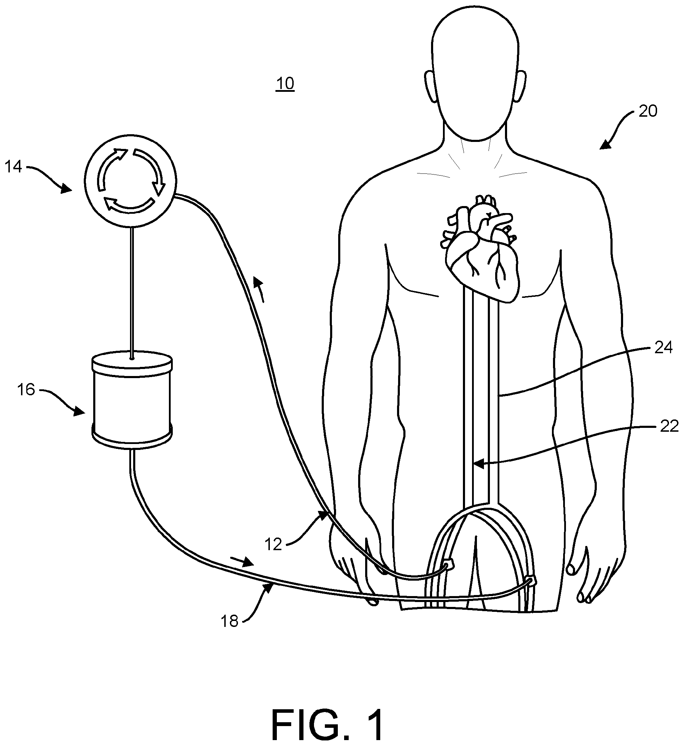

FIG. 1 shows a schematic view of a peripheral venous-arterial extracorporeal membrane oxygenation (VA-ECMO) circulatory support system 10, one of the most frequently used forms of ECMO, operatively coupled to a patient 20. While much of the present disclosure is provided in the context of an ECMO system, the cannule described are useful in other types of extracorporeal circulation systems. In other words, the cannulae and aspects described herein are not limited to use with ECMO systems. As shown in FIG. 1, the system 10 includes a drainage cannula (e.g., a femoral vein cannula) 12, a blood (e.g., centrifugal) pump 14, an oxygenator 16, and a perfusion cannula (e.g., a femoral artery cannula) 18. In operation, when connected to the patient 20, blood is drained from a vein (e.g., the inferior vena cava) 22 of the patient through the drainage cannula 12. The blood is propelled by the blood pump 14 through the oxygenator 16, and oxygenated blood is then returned to an artery (e.g., the femoral artery) which is connected to the aorta 24 of the patient through the perfusion cannula 18. FIG. 1 thus illustrates a peripherally placed VA-ECMO system, which is a configuration commonly employed by cardiologists and other medical professionals in the ECMO procedures.

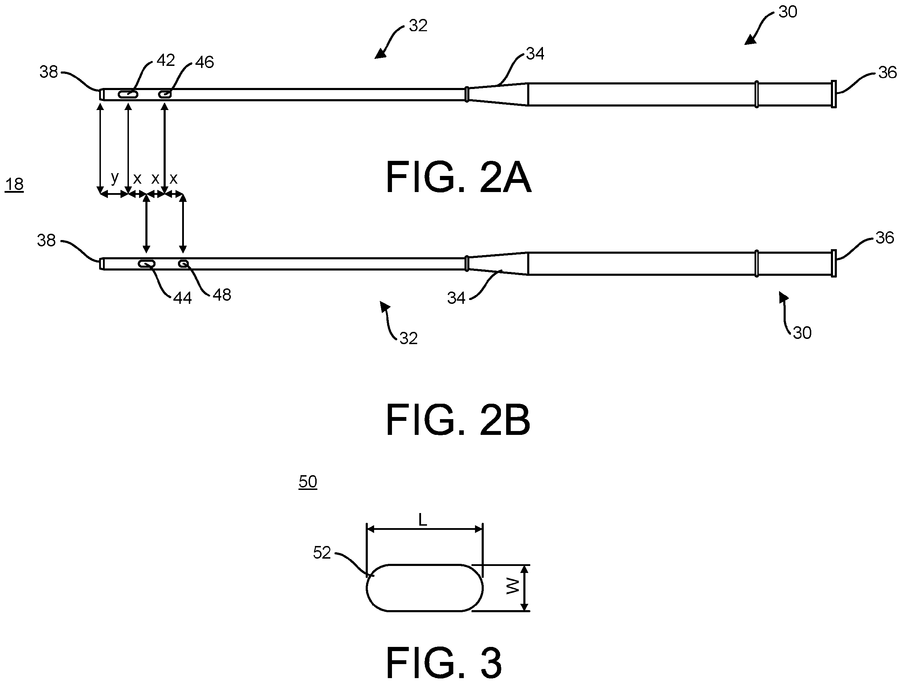

FIG. 2a shows a top side view and FIG. 2b shows a bottom (i.e., 180 degrees apart from FIG. 2a) side view of a perfusion (arterial) cannula 18 according to various embodiments of the present invention. As shown in FIGS. 2a and 2b, the cannula 18 extends from a proximal portion 30 (near the operator) to a distal portion 32 (near the patient's heart). As shown, the proximal and distal portions are coupled by a transition portion 34, where the diameter decreases from a larger proximal diameter to a smaller distal diameter. As explained above, the perfusion cannula 18 is tubular and includes a lumen extending from a proximal opening 36 at the proximal end to a distal tip opening 38 at the distal end. The lumen is adapted to facilitate blood flow therethrough, specifically blood entering through the proximal opening 36 from the extracorporeal circuit (e.g., from the oxygenator) and returning to the patient (e.g., through the distal opening or tip 38).

As shown in FIGS. 2a and 2b, the distal portion further includes a plurality of side holes (i.e., opening) that are coupled to the lumen of the cannula and are sized, shaped and positioned to optimize blood flow as it returns to the patient's artery. As shown, the side holes have a "slotted" configuration, which indicates that the holes have an elongated (i.e., have a length greater than a width) rectangular shape. In some embodiments, the elongated side holes have square corners and in other embodiment the elongated side holes have rounded corners (as shown in FIGS. 2). In some embodiments, all of the side holes present in a cannula have substantially the same size and shape. In certain embodiments, such as those of FIGS. 2, the shape of the side holes are substantially the same, but the sizes differ.

FIG. 3 shows an exemplary slotted side hole 50 according to embodiments of the present invention. As shown, the side hole 50 has a length L and a width W. Also, in the exemplary embodiment of FIG. 3, the side holes have rounded corners. As explained above, in some embodiments, each of the side holes present in a cannula have substantially the same shape and further are characterized by the same ratio between their length L and width W (i.e., L/W ratio). In embodiments, the slotted side holes have an L/W ratio of from about 1.2 to about 3. In various embodiments, the side holes have an L/W ratio from about 1.2 to 2. In some of these it is constant in any given row at the value of 1.5. In other embodiments, the side holes have an L/W ratio of about 2.1 to 3. In some of these it is constant in any given row at the value of about 2.5. In various embodiment, the L and W values for all side holes in a cannula are substantially the same. In other embodiments, the L and W values in adjacent rows are increased moving from row-to-row in the distal direction. In other words, the size of the side holes in at least one row are larger than the size of the side holes in the more proximal rows.

As shown in FIGS. 2, the perfusion cannula 18 includes a plurality of rows of single slotted side holes located at distinct positions along the distal portion 32 of the cannula 18, which together with the distal opening 38, allow blood to exit the cannula 18 and return to the patient 20. As shown, the side holes of these embodiments have rounded corners. As used herein, a "row" of side holes indicates one or more side holes positioned substantially the same distance from the cannula tip 38. As shown in FIGS. 2, the cannula 18 includes four rows of single side holes 42, 44, 46, 48 spaced apart axially along the length of the cannula 18 by a distance X (i.e., the axial midpoints of the associated side holes are spaced apart by a distance X). As further shown, the first side hole row 42 is spaced from the distal tip opening 38 by a distance Y.

As further shown, rows 42 and 46 (shown in FIG. 2a) are circumferentially aligned and are diametrically opposed to rows 44 and 48 (shown in FIG. 2b). While the spacing X shows that the axial spacing of these rows/holes is constant, in other embodiments this spacing varies. In certain embodiments, the value X ranges from about 7 to about 9 mm, and in some embodiments, the spacing X is about 7.5 mm. Also, the first (i.e., distal-most) row 42 axis is shown located at a distance Y from the distal tip 38. In various embodiments, the value Y ranges from about 11 to about 13 mm, and in some embodiments, the spacing Y is about 11.5 mm.

In the exemplary cannula shown in FIGS. 2 the 4 single side slotted hole rows have a ratio L/W constant for any given row 42, 44, 46, 48 at the value of about 2.5. In other embodiments the perfusion cannula may have an L/W ratio ranging from 2.1 to 3. In certain embodiments the L/W ratio may be in the range 2.3 to 2.7. As shown, the side hole 42 is the largest of the side holes and the side holes in each of rows 44, 46 and 48 have a smaller size than that of the adjacent (in a distal direction) row. In other words, the side holes get progressively larger in rows moving from a proximal direction in a distal direction (i.e., toward the distal tip 38). In row 42 (having the largest side holes), the values of L may range from about 6.5 to 8.5 mm, correspondingly the W values range from about 2.6 to 3.4 mm as resulting from an L/W constant ratio of 2.5. In such a cannula, if L in row 42 is equal to 7.5mm, W in the same row is about 3 mm, as provided by a constant L/W ratio of 2.5 (i.e., 7.5/3mm).

In the adjacent rows (moving proximally along the cannula), the length and width values of the side holes in rows 44, 46 and 48 progressively and respectively decrease by constant fractions of length L and width W of the row 42 holes, and in some embodiments, these fractions are equal to L/6 and W/6, so that for a length L value of 7.5mm in row 42, the hole constant length row-to-row decrease is about 1.25mm (i.e. 7.5mm/6) and for a width W value in row 42 of 3mm, the hole constant width row-to-row decrease is about 0.5 mm (i.e., 3mm/6). In some embodiments, the hole length and width values of the row 48 side holes (i.e., the smallest side hole row) are thus reduced by 50% compared to the length and width values of the row 42 side holes.

Similarly, the open area of the side holes in each row progressively decreases by about 30% in rows 44, 46 and 48 as compared to the open area of side holes in the adjacent (more distal) row. In some embodiments, the distal portion of the cannula has a length of about 200 mm and the side holes are concentrated in the first (most distal) fifth of this length (i.e., the most distal 35-40mm of the distal portion), which provides good cannula flexibility and maneuverability during insertion. The relatively larger open areas of side holes in rows 42 and 44, provide for improved lateral delivery of blood flow, which allows the cannula to achieve the goal of attenuating the main flow through the tip (directed toward the heart) with corresponding reduction of the jet stream effect. This reduces stress on blood cells and minimized potential damages to the internal wall of the cannulated vessel.

Moreover, the holes of the two most proximal rows 46 and 48 are dimensioned and positioned in such a way so as to allow a small blood flow also in the direction opposite to the flow delivered by the tip. This secondary flow is sufficient to perfuse the lower limbs and to avoid their ischemia which is a problem in peripheral VA-ECMO due to cannula occupying the entire femoral artery lumen and blocking most interstitial flow towards the legs. In various embodiment, the adjacent holes are on opposing sides of the cannula, so that blood is delivered in a more uniform way along the distal cannula sides, which improves cannula performance and helps keeping the cannula more axially centered inside the vessel.

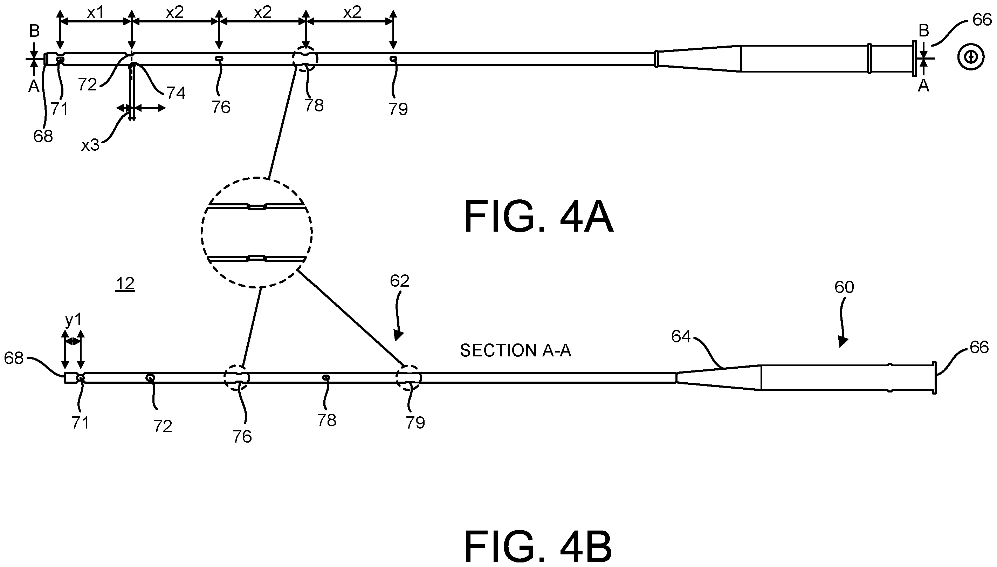

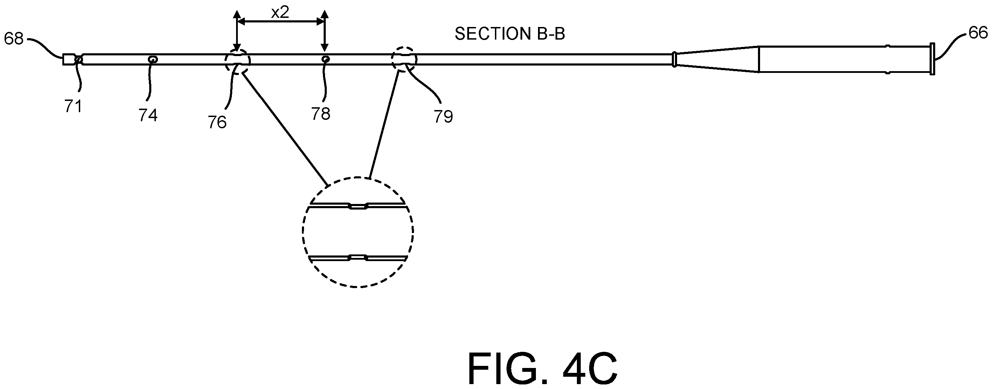

FIGS. 4 show various views of a drainage cannula 12 according to embodiments of the present invention, which may be used in the circulatory support system 10 for draining blood from the patient 20. FIG. 4a shows a perspective view of the cannula 12 and FIGS. 4b and 4c show respectively A-A and B-B sections of FIG. 4a. As shown in FIGS. 4, the cannula 12 extends from a proximal portion 60 (near the operator) to a distal portion 62 (near the patient's heart). As shown, the proximal and distal portions are coupled by a transition portion 64, where the diameter decreases from a larger proximal diameter to a smaller distal diameter. As explained above, the drainage cannula 12 is tubular and includes a lumen extending from a proximal opening 66 at the proximal end to a distal tip opening 68 at the distal end. The lumen is adapted to facilitate blood flow therethrough, specifically blood entering through the distal opening 68 (from a patient vessel) and reaching the extracorporeal circuit (i.e., the centrifugal pump) by traversing the drainage cannula 12 and exiting through the proximal opening 66.

As shown in FIGS. 4, the distal portion further includes a plurality of side holes that are coupled to the lumen of the cannula and are sized, shaped and positioned to optimize blood flow as it is drained from a patient's vein. As shown, the side holes have a "slotted" configuration, which indicates that the holes have an elongated (i.e., have a length L greater than a width W) rectangular shape. In some embodiments, the elongated side holes have square corners and in other embodiment the elongated side holes have rounded corners. In certain embodiments, all of the side holes present in a cannula have substantially the same size and shape, In certain embodiments, the side holes have substantially the same shape, but the sizes are different. As detailed above, FIG. 3 shows an exemplary slotted side hole that can also be used in the embodiments shown in FIGS. 4.

In the exemplary embodiments shown in FIGS. 4, the cannula 12 includes a plurality of rows of slotted side holes located at distinct positions along the distal portion 62 of the cannula 12, which together with the distal opening 68, allow blood to exit the cannula 12 and enter the extracorporeal circuit (i.e., directed to the centrifugal pump). As shown, the side holes of these embodiments have rounded corners. As also used herein, a "row" of side holes indicates one or more side holes positioned substantially the same distance from the cannula tip. As shown in FIGS. 4, the cannula 12 includes six slotted side hole rows 71, 72, 74, 76, 78 and 79 spaced axially along the length of the cannula 12. As shown, the most distal side hole row 71 (i.e., the axial midpoint of the associated side hole) is spaced at a distance Y1 from the distal tip 68 and side hole row 72 (i.e., the axial midpoint of the associated side hole) is spaced proximally at a distance X1 from row 71. Similarly, side hole rows 76, 78 and 79 are all spaced proximally from their adjacent rows by a constant distance X2. Side hole rows 72 and 74 are spaced slightly apart from each other. In other embodiments, the spacing between the various rows is not constant, i.e., each row may be spaced a different amount from each adjacent row. For reference, side hole rows 76, 78 and 79 comprise a first set of side hole rows and side hole rows 72 and 74 comprise a second set of side hole rows.

As shown in FIGS. 4, row 71 (the distal most row) includes four side holes, which are spaced about 90 degrees from each other circumferentially about the cannula 12. Rows 72 and 74 each include a single side hole, the side holes of these adjacent rows spaced about 180 degrees from each other circumferentially. In some embodiments, the holes of rows 72 and 74 are circumferentially aligned with holes of row 71. As shown in FIGS. 4, rows 76, 78 and 79 each have two circumferentially opposed (i.e., spaced about 180 degrees about the circumference of the cannula) side holes. In some embodiments, as shown in FIGS. 4, each pair of opposed holes in row 76, 78 and 79 is alternatively aligned with the opposing side holes of row 71. In the embodiment of FIGS. 4, the distal portion of cannula 12 includes 12 side holes. As shown in FIGS. 4, the side holes or rows 76 and 79 are circumferentially aligned with each other, and the side holes of row 78 are circumferentially offset by about 90 degrees. In the embodiment of FIGS. 4, each of the various rows of the cannula 12 has between one and four slotted side holes.

In FIGS. 4, the ratio L/W of each of the 12 slotted side holes (in 71, 72, 74, 76, 78 and 79) is kept constant at the value of about 1.5. In other embodiments, the L/W ratio of the side holes for venous cannulae may range from about 1.2 to 2. In certain embodiments, the L/W ratio of the side holes ranges from about 1.3 to 1.7. As shown, the side holes of rows 71, 72 and 74 each have the same length L and the same width W. In some embodiments, if the length L of these holes is 4.5mm (ranging from 4 to 5 mm), with an L/W ratio equal to 1.5, the W value is equal to 3 mm. Further, always referring to FIGS 4, for the side hole rows 76, 78 and 79, the lengths and the widths are row-to-row progressively decreased by fixed amounts. In some embodiments the lengths and widths of each of the side holes of rows 76, 78 and 79 are decreased one sixth of the length and width of the 74 single hole row, respectively, moving in a proximal direction along the cannula 12. So, if L in 74 is 4.5 mm and W is 3 mm, the decrease in length is 4.5/6= 0.75 mm and the decrease in width is 3/6=0.5 mm. Therefore in 76 L is equal to 4.5-0,75=3.75 mm and W is 3-0.5=2.5 mm, in 78 L is 3mm and W=2 and in 79 L=2.25mm and W=1.5mm. In other words, the side holes of rows 76, 78 and 79 each get progressively smaller moving in a proximal direction. In these embodiments, the holes of row 79 have a length L and a width W that is reduced by about 50% as compared to the L and W of the holes of rows 71, 72 and 74.

As shown, the axis of row 71 is spaced at a distance Y1 from the tip 68. In various embodiments, the distance Y1 is from about 8 to about 12mm. In some embodiments, the distance Y1 is about 9mm and in other embodiments the distance Y1 is about 10mm. The axis of row 72 is spaced at a distance X1 from the axis of row 71. In some embodiments, the distance X1 is from about 39 to about 43 mm. In some embodiments, the distance X1 is about 41 mm. Further, in some embodiments X1 is about 14 to about 18 mm. In some embodiments the distance X1 is about 16mm.

The axis of row 74 is spaced at a distance X3 of about 3 to 6 mm from the axis of row 72, and in some embodiments, at a distance of 4mm. The axis of rows 76, 78 and 79 are spaced at a distance X2 from the distally adjacent row. In some embodiments, where the overall length of the venous cannula is from about 50 to 70 cm, the distance X2 is from about 48 to 52 mm and, in some embodiments, the distance X2 is about 50 mm. In some embodiments, where the overall length of the venous cannula is from about 25 to 45 cm, the distance X2 is from about 23 to 27 mm and,in some embodiments the distance X2 is about 25mm.

In certain embodiments, about 50% of the length of a typical 350mm distal portion of the cannula is occupied by slotted side holes. Collectively, the side holes provide for a relatively large and well distributed open area, which gives the advantage of reducing blood stagnation, avoiding the blood recirculation. An optimal drainage from the patient is thus achieved.

The above embodiments are only two examples (one per cannula family, either perfusion-arterial or drainage-venous) of the many cannulae that can be realized adopting the claimed structure of slotted side holes and dimensioning the holes within the limits indicated above. Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A cannula for conveying blood in an extracorporeal circulation system, the cannula comprising:
a tubular body having a proximal portion having a first diameter and a distal portion having a second diameter smaller than the first diameter, the tubular body defining an internal lumen extending from a proximal end to a distal end along an axis, the distal end including a distal opening;
wherein the distal portion of the tubular body defines at least two rows of side holes in fluid communication with the internal lumen, a first row disposed proximal to the distal opening and a second row spaced in a proximal direction along the axis from the first row, the side holes of each row having a rectangular shape with a length L and a width W;
wherein the side holes of the first row have a first length L and a first width W and the side holes of the second row have a second length L and a second width W, each of which are less than the first length L and the first width W; and
wherein the L/W ratio of each side hole is between 2.3 and 2.7.

2. The cannula of claim 1 further wherein the first row includes a first side hole and the second row includes a second side hole, each of the first row and second row including only a single side hole.

3. The cannula of claim 1 wherein the first and second rows are spaced apart at a distance of about 7 to 9 mm in the proximal direction.

4. The cannula of claim 2 wherein the first side hole and the second side hole are circumferentially opposed.

5. The cannula of claim 1 further comprising a third row having a third side hole and a fourth row having a fourth side hole, the third row spaced in a proximal direction along the axis from the second row and the fourth row spaced in a proximal direction along the axis from the third rowwherein the side holes of the third row have a third length L and a third width W each of which are less than the second length and the second width W and the side holes of the fourth row have a fourth length L and a fourth width W, each of which are less than the third length L and the third width W.

6. The cannula of claim 5 further wherein each of the first row, second row, third row and fourth row are spaced equally from each other at a distance of from about 7 to about 9 mm.

7. The cannula of claim 5 wherein each of the third row and fourth row include only a single side hole.

8. The cannula of claim 7 wherein the first and third side holes are substantially circumferentially aligned, the second and fourth side holes are substantially circumferentially aligned, and the first and third side holes are generally circumferentially opposed to the second and fourth side holes.

9. The cannula of claim 5 wherein the second length, third length and fourth length are each progressively reduced by about one sixth of the first length and further wherein the second width, third width and fourth width are each progressively reduced by about one sixth of the first width.

10. The cannula of claim 9 wherein the fourth length is about one half the first length and the fourth width is about one half the first width.

11. The cannula of any claim 1 wherein the L/W ratio of each side hole is 2.5.

12. A cannula for conveying blood in an extracorporeal circulation system, the cannula comprising:
a tubular body having a proximal portion having a first diameter and a distal portion having a second diameter smaller than the first diameter, the tubular body defining an internal lumen extending from a proximal end to a distal end along an axis, the distal end having a distal opening;
wherein the distal portion of the tubular body defines a first set of three rows of side holes in fluid communication with the internal lumen, of which a first row is disposed proximal to the distalopening, a second row is spaced in a proximal direction along the axis from the first row, and a third row is spaced in a proximal direction along the axis from the second row, each of the side holes having a rectangular shape with a length L and a width W;
wherein each row of the first set has at least two side holes and wherein the side holes of the first row have a first length L and a first width W and the side holes of the second row have a second length L and a second width W and the side holes of the third row have a third length L and a third width, W the second length and width being less than the first length and width and the third length and width being less than the second length and width;
wherein the L/W ratio of each side hole is between 1.3 and 1.7.

13. The cannula of claim 12 wherein the side holes of each of the first, second and third rows are circumferentially opposed and further wherein each of the side holes of the first row are circumferentially aligned with each of the side holes of the third row.

14. The cannula of claim 13 wherein the first row, second row, and third row are each equally spaced from each other.

15. The cannula of claim 14 wherein each of the first row, second row and third row are equally spaced from each other at a distance of from about 48 to 52 mm.

16. The cannula of claim 14 wherein each of the first row, second row and third row are equally spaced from each other at a distance of from about 23 to 27 mm.

17. The cannula of claim 15 further including a second set of two single side hole rows disposed between the distal opening and the first row of the first set, including a fourth row disposed distal to a fifth row, wherein the fifth row is spaced from the first row of the first set at a distance of from about 44 to 48 mm.

18. The cannula of claim 16 further including a second set of two single side hole rows disposed between the distal opening and the first row of the first set, including a fourth row disposed distal to fifth row, wherein the fifth row is spaced from the first row of the first set at a distance of from about 19 to 23mm.

19. The cannula of claim 18 wherein the side hole lengths of the second set are equal to each other and wherein the side hole widths of the second set are equal to each other and wherein the L/W ratio of the second set side holes is 1.3 to 1.7.

20. The cannula of claim 19 wherein the first length, second length and third length of the first set of side holes are each progressively reduced by about one sixth of the second set length and wherein the first width, second width and third width of the first set of side holes are each progressively reduced by about one sixth of the second side hole width.

21. The cannula of claim 17 wherein fourth row and the fifth row are spaced from each other at a distance of from about 3 to 6 mm.

22. The cannula of claim 12 wherein the L/W ratio of each side hole is 1.5.

23. The cannula of claim 17 further including a sixth row of side holes disposed between the fourth row of the second set and the distal opening.

24. The cannula of claim 23, wherein the sixth row is spaced along the axis in a distal direction from the fourth row at a distance of from about 39 to 43 mm.

25. The cannula of claim 24 where the sixth row of side holes is spaced along the axis in a proximal direction from the distal opening at a distance of from about 8 to 12 mm.

26. The cannula of claim 25 wherein, the fourth row of side holes, the fifth row of side hole and the sixth row of side holes all have the same length and width.

27. The cannula of claim 26, wherein third row of side holes have a length and a width about 50 percent less than the length and width of the fourth, fifth and sixth row of side holes.
